# EUROPEAN PATENT SPECIFICATION

(11) **EP 0 429 109 B1**
(45) Date of publication and mention of the grant of the patent: **28.04.1993**
(21) Application number: 90202786.1
(22) Date of filing: 19.10.1990
(51) Int. Cl.: A61H 23/00, A61H 37/00, A61F 7/00, A61B 8/00

(54) **Apparatus for treating with acoustic waves and diagnosing a patient**
Vorrichtung zum Behandeln und Diagnostizieren eines Patienten mittels akustischen Wellen
Dispositif de traitement et de diagnostic d'un patient par ondes acoustiques

(30) Priority: 14.11.1989 NL 8902809
(43) Date of publication of application: 29.05.1991
(73) Proprietor: Nagy, Lajos Zoltan, NL-1405 ED Bussum (NL)
(72) Inventor: Nagy, Lajos Zoltan, NL-1405 ED Bussum (NL)
(74) Representative: de Vries, Johannes Hendrik Fokke

(56) References cited:
- EP-A- 0 112 082
- WO-A-85/03634
- GB-A- 1 286 016
- US-A- 4 556 070

## Description

The invention relates to an apparatus for treating with acoustic waves or mechanical vibrations and diagnosing a patient, according to the preamble of claim 1.

An apparatus of this type is known from European patent 0 171 423. As described in this patent, such an apparatus is intended for the therapeutic treatment of disturbances in the blood circulation, especially of vaso-constrictions occurring in the limbs of the patient or of body parts with damming which are unsufficiently provided with blood for other reasons. In the specification of this patent it is only noted in short that the treatment is effective when the patient feels warmth at the treated body part, wherein a thermometer can be present which is located against or immediately adjacent the treated body part for measuring the temperature.

GB-A- 1 286 016 discloses an apparatus of the above-mentioned type, wherein the treatment head provides a gentle massaging of the body of a patient. By means of the sensor the massaging is synchronized with the heart activity of the patient.

The invention aims to provide an apparatus of the above-mentioned type, with which said complaints can be treated with an increased effectiveness also in comparison with the described known apparatus.

The invention is based on the insight that the effectiveness of the treatment can not only be determined by measuring the temperature but in a more efficient manner also by means of other parameters of the patient.

In order to improve the effectiveness of the treatment the above-mentioned apparatus is therefore characterized by the characterizing features of claim 1.

Experiments of the applicant have shown that by measuring the change of the heart rate as a result of the treatment the frequency of the generator can be adjusted in a suitable manner in such a manner that a very effective next treatment becomes possible.

It has further been found that a more accurate measurement of the temperature change is obtained if the temperature is measured at several locations on the peripheral ends of the arms or legs of the patient. Accordingly a favourable embodiment of the apparatus according to the invention is characterized in that a plurality, preferably six, temperature sensors are provided, which temperature sensors can be disposed on the peripheral ends of the arms or legs of the patient, wherein the frequency of the generator is adjustable in dependence on the temperature changes as a result of the treatment.

Experiments have further shown that the oxygen concentration in the blood of the patient is affected by the treatment. According to the invention it is therefore advantageous to provide a sensor for measuring the oxygen concentration in the blood of the patient, wherein the frequency of the generator is adjustable in dependence on the change of the oxygen concentration as a result of the treatment.

According to the invention it is possible that the generator is made as a signal generator for generating signals with any desired wave shape and frequency. It has been found that depending on the type of the affection or complaint to be treated, treatment with different wave shapes and frequencies result in an increased effectiveness of the treatment.

According to a preferred embodiment of the invention the treatment head and each sensor are connected to a processing unit which controls the treatment head and processes the measuring signals of the sensor(s) and displays these signals on a display screen, wherein the signal shape and frequency are adjustable through a keyboard.

Thereby the treatment and measurement of the different parameters may occur in an advantageous manner as controlled by a processing unit preferably comprising a microprocessor.

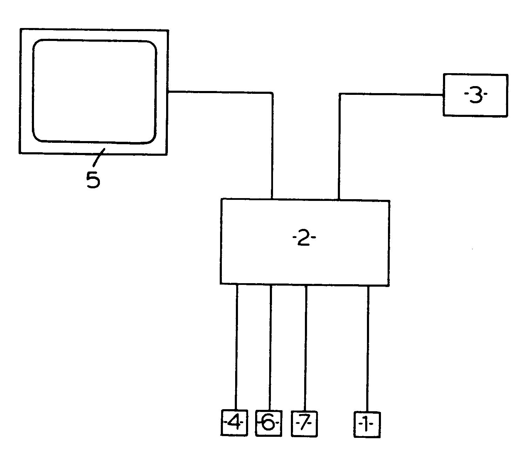

The invention will be further explained by reference to the drawing in which an embodiment of the apparatus according to the invention is shown in a simplified block diagram.

The apparatus shown for treating and diagnosing a patient comprises a treatment head 1 with a sound source not shown. This treatment head 1 can be made in the same manner as the treatment head according to European patent 0 171 423 mentioned above. As an alternative the sound source can be mounted on one end of the cilindrical housing of the treatment head of the described apparatus. It is further noted that instead of a loudspeaker as a sound source it is also possible to use other types of sound sources.

The treatment head 1 is connected to a processing unit 2 which includes a signal generator not further shown, which generator can generate signals with any desired wave shape and frequency. These signals are supplied to the sound source of the treatment head 1. Although for the treatment of many affections of the vein system disturbing the blood circulation, generally a signal with a frequency between 1 and 1000 Hz is used, higher frequencies upto ultrasonic frequencies may be used. Besides square waves it is further possible to use other signal shapes such as more or less sinusoidal signals.

The processing unit controls the treatment head 1 and determines the time during which the body part of the patient is treated with the acoustic waves. Further the processing unit 2 determines the intensity of the acoustic waves. The desired intensity and treatment time can for example be supplied to the processing unit through a keyboard 3.

The medical attendant can determine the different adjustments in accordance with his expertise by means of the results of a previous treatment. Observations have shown that the effectiveness of a treatment can especially be determined by means of the heart rate of the patient. For measuring this heart rate a sensor 4 is provided which supplies the measuring signal to the processing unit 2. The heart rate is measured during a predetermined measuring time before the treatment and during a predetermined measuring time after the treatment. The measuring results obtained can for example be shown on a display screen 5 or in another suitable manner.

Further the temperature of the treated body part, in particular at the peripheral end thereof is of importance. To this end several temperature sensors 6 are provided, preferably six, which for example in case of treating the leg of a patient can be disposed on three different locations on both feet of the patient. The temperature is also measured before and after the treatment.

Further, in the embodiment shown a sensor 7 is provided for measuring the oxygen concentration in the blood. It has been found that the oxygen concentration in the blood is also changed as a result of the treatment of the patient.

All measuring data can be shown in a suitable manner on the display screen 5, wherein in particular the change as a result of the treatment is of importance. The medical attendant can determine the treatment time, intensity, frequency and signal shape through the keyboard for a next treatment. All data relating to a certain patient with respect to the treatment can be stored in a memory not shown.

It is also possible to connect the processing unit 2 through suitable means to a central computer 3, to which a plurality of processing units 2 can be connected and in which all data is stored centrally. Further this central computer may provide adjustment data to the processing units 2.

Although a treatment head with a sound source is used in the above-described embodiment it is noted that the apparatus according to the invention can be also provided with a treatment head with a mechanical vibration source, such as for example a piezo-electric element which is directly placed against the body part to be treated.

The invention is not restricted to the above-described embodiment which can be varied in a number of ways within the scope of the claims.

## Claims

1. Apparatus for treating and diagnosing a patient with acoustic waves or mechanical vibrations, comprising a treatment head (1) with a sound or vibration source, a generator for generating a signal with variable frequency, the treatment head being connectable to the signal generator, a first sensor (4) for measuring the heart rate of the patient and a processing unit connected to the treatment head (1), to the generator and to the first sensor (4), **characterised in that** the processing unit (2) is adapted to measure the heart rate before and after the treatment through said first sensor and by comparing these heart rate measurements controls one or more of the intensity, frequency, treatment time or shape of the signal supplied to the treatment head (1), wherein at least one further sensor is connected to the processing unit (2) for measuring the temperature of the patient.

2. Apparatus according to claim 1, **characterized** in that a plurality, preferably six, temperature sensors are connected to the processing unit (1), which temperature sensors can be disposed on the peripheral ends of the arms or legs of the patient, wherein the processing unit adjusts the frequency of the generator in dependence on the temperature changes as a result of the treatment.

3. Apparatus according to claim 1 or 2, **characterized** in that a sensor (7) for measuring the oxygen concentration in the blood of the patient is connected to the processing unit (2), wherein said processing unit adjusts the frequency of the generator in dependence on the change of the oxygen concentration as a result of the treatment.

4. Apparatus according to anyone of the preceding claims, **characterized** in that the generator is made as a signal generator for generating signals with any desired wave shape and frequency.

5. Apparatus according to anyone of the preceding claims, **characterized** in that the processing unit (2) displays the measuring signals of the sensor(s) on a display screen (5) wherein the signal shape and frequency are adjustable through a keyboard (3).

## Patentansprüche

1. Vorrichtung zum Behandeln eines Patienten und Erstellen einer Diagnose für einen Patienten mit Schallwellen oder mechanischen Schwingungen, mit einem Behandlungskopf (1) mit einer Schall- oder Schwingungsquelle, einem Generator zum Erzeugen eines Signals mit veränderlicher Frequenz, wobei der Behandlungskopf an den Signalgenerator anschließbar ist, einem ersten Sensor (4) zum Messen der Herzfrequenz des Patienten und einer Verarbeitungseinheit, die an den Behandlungskopf (1), den Generator und den ersten Sensor (4) angeschlossen ist, dadurch gekennzeichnet, daß die Verarbeitungseinheit (2) an das Messen der Herzfrequenz durch den ersten Sensor vor und nach der Behandlung angepaßt ist und aufgrund des Vergleichens dieser Herzfrequenzmessungen eine oder mehrere aus der Intensität, Frequenz, Behandlungsdauer oder Form des Signals steuert, das dem Behandlungskopf (1) zugeführt wird, wobei wenigstens ein zusätzlicher Sensor an die Verarbeitungeeinheit (2) zum Messen der Temperatur des Patienten angeschlossen ist.

2. Vorrichtung nach Anspruch 2, dadurch gekennzeichnet, daß eine Mehrzahl, vorzugsweise sechs, Temperatursensoren an die Verarbeitungseinheit (1) angeschlossen sind, welche an den peripherischen Enden der Arme oder der Beine des Patienten angeordnet werden können, wobei die Verarbeitungseinheit die Frequenz des Generators in Abhängigkeit von den als Folge der Behandlung auftretenden Temperaturänderungen einstellt.

3. Vorrichtung nach Anspruch 1 oder 2, dadurch gekennzeichnet, daß ein Sensor (7) zum Messen der Sauerstoffkonzentration in dem Blut des Patienten an die Verarbeitungseinheit (2) angeschlossen ist, wobei die Verarbeitungseinheit die Frequenz des Generators in Abhängigkeit von der als Folge der Behandlung auftretenden Sauerstoffkonzentrationsänderung einstellt.

4. Vorrichtung nach einem der vorhergehenden Ansprüche, dadurch gekennzeichnet, daß der Generator als Signalgenerator zum Erzeugen von Signalen mit jeder gewünschten Wellenform und Frequenz ausgebildet ist.

5. Vorrichtung nach einem der vorhergehenden Ansprüche, dadurch gekennzeichnet, daß die Verarbeitungseinheit (2) die Meßsignale des (der) Sensors(en) in einem Bildschirm (5) anzeigt, wobei die Signalform und -frequenz durch eine Tastatur (3) einstellbar sind.

## Revendications

1. Appareil pour traiter et diagnostiquer un patient à l'aide d'ondes acoustiques ou de vibrations mécaniques, comportant une tête (1) de traitement ayant une source de sons ou de vibrations, un générateur destiné à générer un signal à fréquence variable, la tête de traitement pouvant être connectée au générateur de signal, un premier capteur (4) destiné à mesurer le rythme cardiaque du patient et une unité de traitement connectée à la tête (1) de traitement, au générateur et au premier capteur (4), caractérisé en ce que l'unité (2) de traitement est destinée à mesurer le rythme cardiaque avant et après le traitement par l'intermédiaire dudit premier capteur et, en comparant ces mesures du rythme cardiaque, commande un ou plusieurs des paramètres constitués par l'intensité, la fréquence, le temps de traitement et la forme du signal fourni à la tête (1) de traitement, au moins un autre capteur étant connecté à l'unité (2) de traitement pour mesurer la température du patient.

2. Appareil selon la revendication 1, caractérisé en ce que plusieurs, avantageusement six, capteurs de température sont connectés à l'unité (1) de traitement, lesquels capteurs de température peuvent être disposés sur les extrémités distales des bras ou des jambes du patient, l'unité de traitement réglant la fréquence du générateur en fonction des variations de température par suite du traitement.

3. Appareil selon la revendication 1 ou 2, caractérisé en ce qu'un capteur (7) destiné à mesurer la concentration d'oxygène dans le sang du patient est connecté à l'unité (2) de traitement, ladite unité de traitement réglant la fréquence du générateur en fonction de la variation de la concentration d'oxygène par suite du traitement.

4. Appareil selon l'une quelconque des revendications précédentes, caractérisé en ce que le générateur est réalisé en tant que générateur de signaux destiné à générer des signaux ayant toutes formes d'ondes et fréquences souhaitées.

5. Appareil selon l'une quelconque des revendications précédentes, caractérisé en ce que l'unité (2) de traitement affiche les signaux mesurés du ou des capteurs sur un écran d'affichage (5), la forme et la fréquence des signaux pouvant être réglées par l'intermédiaire d'un clavier (3).
